# EUROPEAN PATENT SPECIFICATION

(11) **EP 2 720 674 B1**
(45) Date of publication and mention of the grant of the patent: **22.07.2020**
(21) Application number: 12730856.7
(22) Date of filing: 15.06.2012
(51) Int. Cl.: A61K 8/58, A61Q 15/00, A61Q 19/00, A61K 8/88, A61K 8/81, A61K 31/695, A61K 31/785, A61P 17/00

(54) **USE OF A COMPOUND COMPRISING AT LEAST A NUCLEOPHILE FUNCTION FOR CAPTURING CARBONYL COUMPOUND RESULTING FROM THE REACTION BETWEEN ONE OR MORE COUMPOUNDS CONSTITUTING SEBUM AND OZONE**
VERWENDUNG EINES MITTELS ENTHALTEND MINDESTENS EINE NUCLEOPHILE FUNKTION ZUR EINFANG VON KOMPONENTEN STAMMEND AUS DIE REAKTION ZWISCHEN EINER ODER MEHREREN KOMPONENTEN AUS TALG ODER OZON
UTILISATION D'UN COMPOSÉ COMPRENANT AU MOINS UNE FONCTION NUCLÉOPHILE POUR CAPTURER LES COMPOSÉS DE CARBONYLE RÉSULTANT DE LA RÉACTION ENTRE UN OU PLUSIEURS COMPOSÉS CONSTITUANT LE SÉBUM ET L'OZONE

(30) Priority: 16.06.2011 FR 1155291; 29.11.2011 US 201161564727 P
(43) Date of publication of application: 23.04.2014
(73) Proprietor: L'OREAL, 75008 Paris (FR)
(72) Inventor: SAMAIN, Henri, F-91570 Bievres (FR)
(74) Representative: Martin-Charbonneau, Virginie
(86) International application number: PCT/EP2012/061457
(87) International publication number: WO 2012/172057

(56) References cited:
- EP-A1- 1 254 652
- EP-A2- 0 118 180
- FR-A1- 2 783 164
- US-A- 5 954 869
- US-A1- 2007 104 660
- US-A1- 2009 293 899
- A. WISTHALER ET AL: "Atmospheric Chemistry Special Feature: Reactions of ozone with human skin lipids: Sources of carbonyls, dicarbonyls, and hydroxycarbonyls in indoor air", PROCEEDINGS OF THE NATIONAL ACADEMY OF SCIENCES, vol. 107, no. 15, 13 April 2010 (2010-04-13), pages 6568-6575, XP055024831, ISSN: 0027-8424, DOI: 10.1073/pnas.0904498106

## Description

The invention relates to a process for capturing one or more carbonyl compounds B resulting from the reaction between one or more compounds constituting sebum and ozone on the surface of a material or in a material, in which one or more compounds A having one or more nucleophilic functions F_{A} forming in situ on the surface of said material or in the material a layer capturing said compound(s) B is (are) applied to the surface of said material.

The invention also relates to the cosmetic use of one or more compounds A having one or more nucleophilic functions F_{A} capable of reacting with one or more carbonyl compounds B resulting from the reaction between one or more compounds constituting sebum and ozone, as a soothing agent, said compound(s) A being intended for preventing and/or treating discomforting reactions of keratin materials induced by the carbonyl compounds B.

The invention also relates to the cosmetic use of one or more compounds A having one or more nucleophilic functions F_{A} capable of reacting with one or more carbonyl compounds B resulting from the reaction between one or more compounds constituting sebum and ozone, as an agent for reducing or even eliminating the unpleasant body odours produced by the carbonyl compounds B.

Generally, keratin materials such as the skin, the hair or the scalp are directly in contact with the oxidizing agents found in the environment, which can make them particularly sensitive.

Indeed, the sources of oxidizing agents in the environment include in particular oxygen activated by ultraviolet radiation and also, in polluted or non-polluted air, ozone, nitrogen oxides and sulphur oxides. The ozone concentration may, moreover, prove to be particularly high, including in enclosed spaces, because of atmospheric pollution and the presence of many electrical devices in everyday life.

Such oxidizing agents react with keratin materials and produce, for the most part, oxidized compounds which are neutralized by the natural antioxidant systems. However, ozone in particular can react with keratin materials by means of reactions which in particular take place at the surface of the outermost layer of the skin, the hair or the scalp, and the reaction products that are formed following these reactions can cause irritations or discomforting reactions on the skin, the hair and/or the scalp, which can in particular be reflected by red patches, itching, hot and/or burning sensations, stinging and tautness.

In particular, the secretion by the sebaceous glands of a lipid film called "sebum" at the surface of the skin, the hair or the scalp makes it possible in particular to protect them against the action of ozone. It has been observed that the reaction of ozone with the unsaturated lipids constituting sebum at the surface of keratin materials resulted in breaking of the lipid chains at their double bonds and in the formation of aldehyde compounds, i.e. in compounds which have one or more aldehyde functions in their structure.

This is thus the case of the compounds hexanal, octanal, noneal, nonanal, decanal, undecanal and dodecanal as regards monoaldehydes.

The same is true for 1,4-butanedial as regards dialdehydes.

The aldehyde compounds may also contain, in addition to the aldehyde function(s), other functions, such as ethylene (6-methyl-S-hepten-2-one) or ketone (4-oxopentanal) or acid (4-oxobutanoic acid) functions (cf. respectively below).

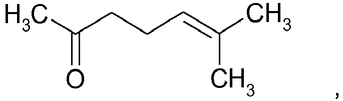

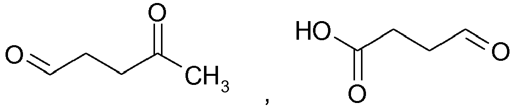

Other compounds with carbonyl functions result from these reactions, it being possible for said compounds, despite the absence of aldehyde functions, to be reactive and to cause such discomforting reactions. This is the case for certain ketones, bearing, in addition to the ketone function, a second group that activates this ketone, in particular at least one hydroxyl function. This is the case for the compound 1-hydroxy-6-methylhepten-2-one, represented below, for example:

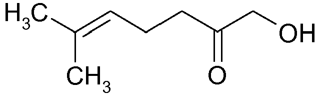

Once formed, these carbonyl compounds cause irritations or discomforting reactions on the skin, the hair and/or the scalp.

Furthermore, the carbonyl compounds resulting from the reaction of ozone and of the unsaturated lipids constituting sebum are often odorous entities, such as nonanal or nonenal, which possibly result in unpleasant body odours being given off.

They may also change and, via oxidation, for example, produce unpleasant body odours. This is the case for 1,4-butanedial, which can, via oxidation, give 1,4-butanedioic acid.

Such reactions can occur both on the skin and on the hair, or even the clothing, soiled by said sebum. Indeed, the molecules constituting the sebum present in the hair can react with ozone and form carbonyl compounds which migrate to the skin.

Among the unsaturated lipids constituting sebum, squalene reacts with ozone in order to form acetone, which is a volatile compound, and 4-oxopentanal which will penetrate into the epidermis and cause irritations or discomforting reactions.

Moreover, the higher the ozone concentration in the air, the more the skin secretes lipid compounds such as squalene, which causes the formation close to the skin of numerous aldehyde compounds, thus increasing the problems of skin discomfort. Given that squalene can be found in sebum in relatively large amounts, which can range up to 12% by weight, the concentration of aldehyde compounds formed may also prove to be high.

It is not possible, in order to combat the formation of these carbonyl compounds, to eliminate or reduce the amount of sebum secreted by the sebaceous glands since, in this case, the skin would no longer be protected against the action of ozone, nor is it possible to add squalene in order to promote the reaction with ozone, since the concentration of aldehyde compounds formed would not be any the less because of this.

Furthermore, the use of conventional antioxidants, such as ascorbic acid, tocopherols, tocotrienols, ubiquinones or glutathione, is not satisfactory for combating the formation of the aldehyde compounds.

Application US-A-2007/104660 discloses an amino silane for use in oral care, US-A-2009/293899 and FR-A-2783164 hair treatment compositions containing an amino silane, US-A-5,954,869 aminosilane as antimicrobial additive for food or cosmetics, EP-A-1254652 a skin cleaning product for removing keratotic plugs, from skin surfaces, with poly(vinyl acetamide), and EP-A-0118180 skin and hair care products for alleviating seborrheic conditions comprising a silanized adsorbent.

There is therefore a real need to implement, on keratin materials or materials in contact with keratin materials, a cosmetic treatment process capable of satisfactorily blocking the carbonyl compounds resulting from the reaction between the molecules constituting sebum and ozone, in order to minimize, or even eliminate, the discomforting reactions which are caused by these carbonyl compounds and also the unpleasant body odours that can be produced by these same carbonyl compounds.

The applicant has discovered, surprisingly, that by bringing one or more carbonyl compounds B resulting from the reaction between one or more components constituting sebum into contact with one or more compounds A having one or more nucleophilic functions F_{A}, it is possible to block said compounds B by forming a "capture" layer which prevents the compounds B from migrating to any material that may be in contact with the latter.

In particular, by applying to keratin materials, in particular the skin and/or the hair, a cosmetic composition comprising, in a cosmetically acceptable medium, one or more compounds comprising one or more nucleophilic functions capable of reacting with one or more carbonyl compounds resulting from the reaction between one or more compounds constituting sebum and ozone, it is possible to block the migration of the carbonyl compounds to human keratin materials and to minimize, or even eliminate, the irritations and discomforting reactions and also unpleasant body odours that may be produced by these carbonyl compounds.

Moreover, the applicant has also noted that, by applying particular compounds having one or more nucleophilic functions which are capable of condensing *in situ* in a material and in particular in the superficial layers of the skin, it is possible to form a condensate which confers, on the material and in particular on the skin, a property of capturing the carbonyl compounds resulting from the reaction between the compounds constituting sebum and ozone. This "capture" layer is therefore formed by condensation *in situ* and is capable of capturing the carbonyl compounds, in particular aldehyde compounds, in order to prevent their migration into human keratin materials, in particular the epidermis. More particularly, the capture layer obtained is formed in the zone of the stratum corneum.

In other words, the nucleophilic functions of these compounds applied to keratin materials such as the skin make it possible to capture the carbonyl compounds resulting from the reaction between ozone and the molecules constituting sebum. The nucleophilic function(s) of these compounds is (are) also called capture functions.

Moreover, the applicant has also noted that the application of particular compounds having one or more nucleophilic functions which exhibit a strong affinity with keratin also makes it possible to block the carbonyl compounds by forming a layer on the surface of the keratin materials. The layer thus formed makes it possible to prevent the migration of the carbonyl compounds on human keratin materials.

In other words, the applicant has brought to the fore that applying compounds having one or more nucleophilic functions which are either capable of condensing *in situ* in the superficial layers of the skin so as to form an internal capture layer, or capable of forming an external capture layer on the surface of a keratin material such as the skin, makes it possible to block the carbonyl compounds formed following the effect of ozone on the lipids constituting sebum.

The present invention relates to a process for capturing one or more carbonyl compounds B resulting from the reaction between one or more compounds constituting sebum and ozone on the surface of a material or in a material, in which one or more compounds A having one or more nucleophilic functions F_{A} is (are) applied to the surface of said material so as to form, in situ on the surface of said material or in said material a layer capturing said compound(s) B.

The invention also relates to the cosmetic use of one or more compounds A having one or more nucleophilic functions F_{A} capable of reacting with one or more carbonyl compounds B resulting from the reaction between one or more compounds constituting sebum and ozone, as a soothing agent, said compound(s) A being intended for preventing and/or treating discomforting reactions of keratin materials induced by said carbonyl compounds B.

More particularly, the compounds A having one or more nucleophilic functions F_{A} capable of reacting with one or more carbonyl compounds B are used, as a soothing agent, said compound(s) A being intended for treating discomforting reactions of keratin materials induced by said carbonyl compounds B.

For the purpose of the present invention, the term "material" is intended to mean keratin materials or materials in contact with said keratin materials.

The term "keratin material" is intended to mean the skin (face, body, lips, scalp), the hair, the eyelashes, the eyebrows, the nails and the mucous membranes.

In particular, the materials in contact with a keratin material may be selected from fabrics and leather and may correspond to any material that may be in contact with keratin materials.

More particularly, the material is a keratin material and the compounds A are contained in a cosmetic composition containing a cosmetically acceptable medium.

The term "carbonyl compound" is intended to mean any organic molecule comprising in its structure one or more carbonyl functions: -C=O and optionally, in addition, one or more other functions selected in particular from acid, hydroxyl and ethylene functions. The carbonyl compounds generally comprise aldehydes and ketones and the tautomeric forms thereof.

In particular, the carbonyl compounds comprise one or more aldehyde functions and, even more particularly, the carbonyl compounds are aldehyde compounds.

According to the present invention, the term "discomforting reaction" is intended to mean all of the discomforts of the skin and/or of the hair and/or of the scalp that are caused by the carbonyl compounds resulting from the reaction between one or more compounds constituting sebum and ozone and that in particular take the form of red patches, itching, hot and/or burning sensations, stinging or tautness.

According to the invention, the term "soothing agent" is intended to mean an agent which helps to reduce the discomfort of keratin materials caused by the carbonyl compounds resulting from the reaction between one or more compounds constituting sebum and ozone, by soothing the sensations previously mentioned.

The invention also relates to the cosmetic use of one or more compounds A comprising one or more nucleophilic functions F_{A} capable of reacting with one or more carbonyl compounds B resulting from the reaction between one or more compounds constituting sebum and ozone, as an agent for reducing or even eliminating the unpleasant body odours produced by the carbonyl compounds B.

According to another aspect, the present invention relates to other visible signs that may also appear, or keratin disorders, in particular skin disorders, this time advantageously requiring a dermatological treatment.

The invention also relates to compounds A having one or more nucleophilic functions F_{A} capable of reacting with one or more carbonyl compounds B resulting from the reaction between one or more compounds constituting sebum and ozone, as a dermatological agent for preventing and/or treating keratin disorders, and in particular skin disorders, induced by said carbonyl compounds B.

More particularly, the invention deals with compounds A having one or more nucleophilic functions F_{A} capable of reacting with one or more carbonyl compounds B resulting from the reaction between one or more compounds constituting sebum and ozone, as a dermatological agent for treating keratin disorders, and in particular skin disorders, induced by said carbonyl compounds B.

According to the present invention, the term "keratin disorder" is intended to mean all the signs on keratin materials, caused by irritation, inflammation and/or allergy reactions, which can materialize in particular through the appearance of dry patches, oedema and/or spots, inflammatory erythema, cutaneous atopy, atopic dermatitis, urticaria, eczema, seborrhoeic dermatitis and/or inflammatory hyperpigmentations.

According to the invention, the term "preventing" or "prevention" is intended to mean reducing the risk of occurrence or slowing down the occurrence of a given phenomenon, namely, according to the first aspect of the present invention, the effect of discomfort produced by the skin reactions mentioned above or the skin disorders as defined above.

Other subjects and characteristics, aspects and advantages of the invention will emerge even more clearly on reading the description and the examples that follow.

According to a first aspect of the present invention, the compounds A are capable of condensing *in situ* and contain one or more nucleophilic functions F_{A} capable of reacting with one or more compounds B comprising one or more carbonyl functions resulting from the reaction of one or more constituents of sebum with ozone.

In particular, the compound(s) A is (are) capable of condensing *in situ* in the keratin material, in particular in the superficial layers of the skin, in particular the zone of the stratum corneum.

According to a first embodiment of this first aspect, the compound A comprises at least two reactive functions: a function which makes it possible for the compound A to condense with itself and at least one nucleophilic function F_{A} also called "capture function", or one function which results in the formation of a nucleophilic function F_{A} after condensation.

According to a second embodiment of this first aspect, a group of compounds A is used. This embodiment comprises the use of at least two molecules capable of condensing with one another. In this case, and according to the invention, the molecules constituting the group of compounds must comprise one (or more) function(s) making possible condensation of the group A, and one (or more) function(s) making possible the presence of a nucleophilic function F_{A} after condensation. According to one particular embodiment, the nucleophilic function(s) F_{A} is (are) present on only one of the compounds of the group of compounds A. In another embodiment, the nucleophilic function(s) F_{A} is (are) present on at least two compounds of the group of compounds.

In other words, with a group of compounds A consisting of the molecules A1 and A2, it is possible for A2 not to contain a nucleophilic function F_{A}. In this case, either the compound A1 exhibits nucleophilic functions F_{A}, or the latter appear on conclusion of the condensation reaction. Alternatively, the compound A2 may contain nucleophilic functions F_{A}. In this case, the compound A1 may contain no nucleophilic function F_{A}.

By way of illustration, the condensation of the group of compounds A can be represented in the following way.

Group of compounds A comprising two compounds: A1 and A2
- 1^{st} case: A1 comprises a nucleophilic function (compound F_{A}-A1); not A2: condensation product: 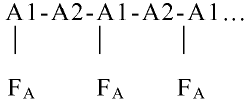
- 2^{nd} case: A1 and A2 comprise a capture function, in each case identical or different, (compound F_{A}-A1 and compound F'_{A}-A2): condensation product:

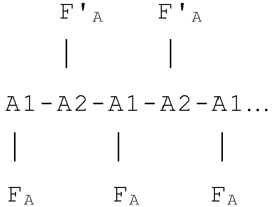

It is understood that this particularly simplified schematic example is presented only by way of illustration and should not be regarded as limiting the invention.

The condensation and nucleophilic functions will be described subsequently with reference to A, which will denote without distinction a compound A or a group of compounds A (for example A1 + A2) described above.

The nucleophilic function(s) must be at least in part free after condensation of A in order to be able to react with the carbonyl compounds. Alternatively, the nucleophilic function(s) F_{A} may not exist or may not be in free form in the compound or group of compounds A, but may appear or become free on completion of the condensation thereof. They may, for example, be primary amine functions which might appear subsequent to the action of an enzyme naturally present on the skin.

Preferably, the nucleophilic or capture functions F_{A} which are free after condensation must be present in a proportion such that F_{A}/A > 0.1 and preferentially > 0.5, F_{A} and A respectively representing the amounts by number of nucleophilic functions which are free after condensation and of molecules of A. In other words, there exists, after condensation, at least one free capture function per 10 molecules of A, preferably at least 5 free capture functions.

The nucleophilic functions F_{A} of the compound or group of compounds A are typically selected from amines and the other nucleophilic functions, such as hydroxyl, thiol, sulphate and phosphate functions. Preferably, they are amines; preferentially, primary amines.

The condensation of A can originate from the reaction of a nucleophilic entity with an electrophilic entity. According to a first alternative, the condensation functions are typically those which make possible the creation of a bond by elimination of a water molecule, according to the following equation:

R-OH + HO-R' →R-O-R' + H₂O

In one specific embodiment, the compounds employed are organosilanes.

According to a second alternative, the condensation can originate from the reaction of an entity by pulling a proton off a second entity, in particular according to one of the following equations:

R-CH=CH₂ + H-R' → R-CH₂-CH₂-R'

and

R-CH=NH + H-R' → R-CH₂-NH-R'

In the above equations, R and R' denote, independently of one another, any groups of atoms, it being understood that the product of the condensation of A must comprise at least one free nucleophilic function F_{A}.

Preferably, the condensation takes place according to the first alternative described above.

In one specific embodiment, the compound(s) A is (are) selected from organic silicon compounds.

In particular, the organic silicon compounds comprise from 1 to 3 silicon atoms and at least two hydroxyl or hydrolysable groups per molecule. The hydrolysable groups are preferably alkoxy, aryloxy or halogen groups.

The compound A is selected from organosilanes comprising a silicon atom and organosiloxanes comprising two or three silicon atoms, preferably two silicon atoms.

According to one preferred embodiment, the compound A is an organosilane. The compound A can in particular be an alkoxysilane and preferably a functionalized alkoxysilane.

The group of compounds A can be the mixture of an organic silicon compound as described above (compound A1) and of at least one other compound (compound A2) such that the group comprising the organic silicon compound and the compound A2 is capable of condensing in situ.

Preferably, the compound(s) is (are) selected from the compounds of formula (I): 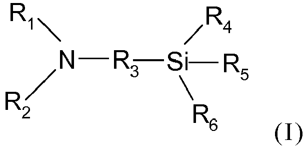 in which:
R₄ represents a halogen or a group OR' or R'₁;
R₅ represents a halogen or a group OR" or R'₂;
R₆ represents a halogen or a group OR'" or R'₃;
R₁, R₂, R₃, R', R", R"', R'₁, R'₂ and R'₃ represent, independently of one another, a saturated or unsaturated, linear or branched hydrocarbon-based group, optionally bearing additional chemical groups, R₁, R₂, R', R" and R'" also possibly denoting hydrogen, and at least two of the groups R₄, R₅ and R₆ denoting, respectively, OR', OR" et OR''', at least two of the groups R', R" and R'" being other than hydrogen.

Preferably, the groups R₁, R₂, R', R'₁, R'₂, R'₃, R"and R'" are selected from C₁-C₁₂ alkyl, C₆ to C₁₄ aryl, C₁ to C₈ alkyl-C₆ to C₁₄ aryl, and C₆ to C₁₄ aryl-C₁ to C₈ alkyl radicals.

According to another specific embodiment, the orgnosiloxane(s) used in the composition according to the invention is (are) selected from the compounds of formula (II): 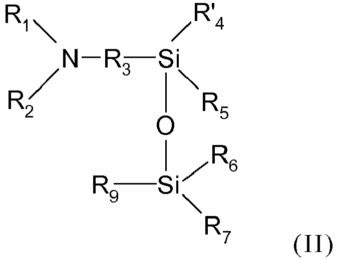 in which:
R₁, R₂, R₃, R₅ and R₆ are defined as above;
R'₄ represents a halogen atom or a group OR₁₁;
R₇ represents a halogen atom or a group OR₁₀ or R"₁;
R₉ represents a halogen atom or a group OR₈, R"₂ or R₃NR₁R₂;
R"₁, R"₂, R₈, R₁₀ and R₁₁ represent a linear or branched, saturated or unsaturated hydrocarbon-based group optionally bearing additional chemical groups, the groups R₁₁, R₁₀ and R₈ also possibly representing a hydrogen atom; at least one of the groups R₆, R₇, and R₉ denoting a halogen atom or a group OR''', OR₁₀ or OR₈.

Preferably, the groups R"₁, R"₂, R₈ or R₁₀ and R₁₁ are selected from C₁-C₁₂ alkyl, C₆ to C₁₄ aryl, C₁ to C₈ alkyl-C₆ to C₁₄ aryl, et C₆ to C₁₄ aryl-C₁ to C₈ alkyl radicals.

In particular, the halogen atom is a chlorine atom.

The organic silicon compound(s) used in the composition according to the invention is (are) preferably organosilanes selected from the compounds of formula (III): 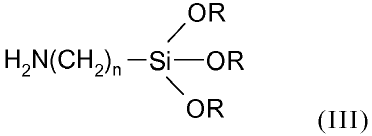 in which the radicals R, which may be identical or different, are selected from C₁-C₆, preferably C₁-C₂, alkyl radicals and n is an integer from 1 to 6, preferably from 2 to 4.

Particularly preferably, the organic silicon compound present in the composition according to the invention is γ-aminopropyltriethoxysilane, referred to as APTES in the rest of this text, or a derivative thereof.

The organic silicon compound(s) may be present in the cosmetic composition according to the invention in a content ranging from 0.001 to 50% by weight, preferably in a content by weight ranging from 0.01% to 20% by weight, relative to the total weight of the composition.

The compound can be applied in combination with other aminated or non-aminated silane monomers, in particular with a molecule having the ability to form an Si-O-Si bond. By way of illustration, mention may be made of methyltriethoxysilane (MTES).

A second compound capable of reacting with some of the capture functions can also be applied at the same time or in a second step.

This may be, for example, an aldehyde or DHA, or an ionic and preferentially anionic and preferentially multianionic compound.

This second compound consolidates the wear property of the capture layer.

In accordance with this first aspect of the present invention, the organic silion compounds condense *in situ* in the superficial layers of the skin, in particular in the zone of the stratum corneum.

In particular, the organic silicon compounds condense *in situ* in the zone of the stratum corneum so as to form a capture layer. This layer is not necessarily continuous but can be composed of a multitude of connected domains. Thus, the carbonyl compounds B formed at the surface of the skin following the reaction between one or more compounds constituting sebum and ozone will react with the product of the condensation of the compounds A, by means of the nucleophilic or capture functions F_{A} which are free after condensation.

Preferably, the nucleophilic functions F_{A} of the compounds A will form one or more covalent bonds with the carbonyl functions of the compounds B. In this way, the carbonyl compounds B will be blocked by the capture layer in the zone of the stratum corneum.

According to a second aspect of the present invention, the cosmetic composition comprises one or more compounds A comprising one or more nucleophilic functions F_{A} which exhibit an affinity with the surface of a keratin material.

For the purpose of the present invention, the term "affinity" with the surface of a keratin material is intended to mean that the compounds A in accordance with this aspect of the present invention do not penetrate into the keratin material, but remain attached at its surface.

Thus, the compounds A comprising one or more nucleophilic functions F_{A} attach to the surface of the keratin materials. In particular, the compound(s) A attach to the surface of the skin without penetrating into the superficial layers of the skin, in particular into the zone of the stratum corneum.

In this way, the compounds A are capable of forming an external capture layer on the surface of a keratin material.

Preferentially, these compounds A comprise at least one nucleophilic function F_{A} of the non-quaternized amine, preferably primary amine, type. In particular, it is a polymer bearing pendant amino groups, more particularly a polymer bearing at least 10% (relative to the monomeric entities) of amino functions.

In particular, it is a polymer bearing at least 10% (relative to the monomeric entities) of amino functions.

In particular, they result from the partial hydrolysis of polyvinylformamide, acetamide, etc.

In particular, they are polyvinylformamides hydrolysed to degrees ranging from 5% to 50%.

In accordance with the second aspect of the present invention, the compound(s) A is (are) selected more particularly from vinylformamide/vinylformamine copolymers comprising:
from 10 to 95 mol% of units of the following formula (IV): 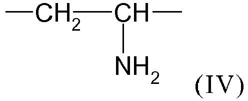
and from 90 to 5 mol% of units of the following formula (V):

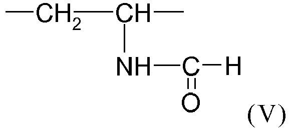

The vinylformamide/vinylformamine copolymer(s) which can be used in the compositions according to the invention preferably comprise(s) from 10 to 60 mol% of units of formula (IV) and more particularly from 20 to 40 mol%.

The vinylformamide/vinylformamine copolymer(s) according to the invention preferably comprise(s) from 30 to 90 mol% of units of formula B and more particularly from 60 to 80 mol%.

The copolymers according to the invention can be obtained, for example, by partial hydrolysis of polyvinylformamide. This hydrolysis can be carried out in an acidic or basic medium.

The vinylformamide/vinylformamine copolymer(s) according to the invention can optionally comprise one or more additional monomer units. In this case, the latter preferably represent less than 20 mol% of the copolymer.

According to a preferred embodiment, the vinylformamide/vinylformamine copolymer(s) according to the invention consist(s) solely of units of formula (IV) and of units of formula (V).

The weight-average molecular weight of said copolymer, measured by light diffraction, can vary from 10 000 to 30 000 000 g/mol, preferably from 40 000 to 1 000 000 g/mol and more particularly from 100 000 to 500 000 g/mol.

The cationic charge density of said copolymer can vary from 2 meq/g to 20 meq/g, preferably from 2.5 to 15 meq/g and more particularly from 3.5 to 10 meq/g.

Mention may be made, as example of vinylformamide/vinylformamine copolymers which can be used in the compositions according to the invention, inter alia, of the products sold under the name Lupamin by BASF, such as, for example, and without limitation, the products provided under the names Lupamin 9030 and Lupamin 9010.

The vinylformamide/vinylformamine copolymer(s) is (are) present in the compositions according to the invention in proportions preferably ranging from 0.001% to 40% by weight, more preferentially from 0.01% to 6% by weight and more particularly from 0.1% to 3% by weight, relative to the total weight of the composition.

In accordance with this second aspect of the present invention, the vinylformamide/vinylformamine copolymers attach to the surface of the skin in such a way that their nucleophilic functions F_{A}, namely their amine, preferably primary amine functions, react with the carbonyl compounds B.

Generally, the compounds A in accordance with the invention are present in a composition comprising a cosmetically acceptable medium.

The term "cosmetically acceptable medium" is intended to mean a medium compatible with the skin and/or its appendages, which has a pleasant colour, odour and feel and which does not cause any unacceptable discomfort (stinging, tautness or red patches) liable to discourage the consumer from using this composition.

Preferably, the cosmetically acceptable medium comprises water and/or one or more cosmetically acceptable solvents selected from C₁-C₄ lower alcohols, such as ethanol, isopropanol, tert-butanol or n-butanol, polyols such as propylene glycol, polyol ethers, C₅-C₁₀ alkanes, C₃-C₄ ketones such as acetone and methyl ethyl ketone, C₁-C₄ alkyl acetates such as methyl acetate, ethyl acetate and butyl acetate, dimethoxyethane, diethoxyethane, and mixtures thereof.

The cosmetic composition may also comprise one or more conventional cosmetic adjuvants selected from antioxidants, UV screening agents, emollients, moisturizers, biological active agents, anti-acne active agents, cationic, amphoteric, non-ionic and anionic polymers, powders, peroxides, sebum-regulating agents such as retinoic compounds, anti-seborrhoeic agents, fillers, peracids, zinc carboxylic salts, basifying or acidifying agents or any other ingredient normally used in the cosmetics and/or dermatological field.

Among the antioxidants, mention may be made especially of tocopherol and esters thereof, in particular tocopheryl acetate; ferulic acid; serine; ellagic acid, phloretin, polyphenols, tannins, tannic acid, epigallocatechin and natural extracts containing same, anthocyans, rosemary extracts, olive leaf extracts, for instance those from the company Silab, green tea extracts, resveratrol and derivatives thereof, ergothioneine, N-acetylcysteine, an extract of the brown alga *Pelvetia canaliculata,* for instance Pelvetiane® from Secma, chlorogenic acid, biotin, chelating agents, such as BHT and BHA, N,N'-bis(3,4,5-trimethoxybenzyl)ethylenediamine and salts thereof; idebenone, plant extracts, for instance Pronalen Bioprotect™ from the company Provital; coenzyme Q10, bioflavonoids, SODs, phytantriol, lignans, melatonin, pidolates, glutathione, caprylyl glycol, phloretin, Totarol™ or extract of *Podocarpus totara* containing Totarol (totara-8,11,13-trienol or 2-phenanthrenol, 4b,5,6,7,8,8a,9,10-octahydro-4b,8,8-trimethyl-1-(1-methylethyl)-; a jasmine extract such as the product sold by Silab under the name Helisun®; hesperitin laurate such as Flavagrum PEG® from the company Engelhard Lyon; an extract of *Paeonia suffruticosa* root, such as the product sold by the company Ichimaru Pharcos under the name Botanpi Liquid B®; an extract of lychee such as the extract of lychee pericarp sold by the company Cognis under the name Litchiderm LS 9704®, an extract of pomegranate fruit (*Punica granatum*), such as the product sold by the company Draco Natural Products.

The organic screening agents are selected in particular from dibenzoylmethane derivatives, anthranilates; cinnamic derivatives; salicylic derivatives; benzylidenecamphor derivatives; benzophenone derivatives; β,β-diphenylacrylate derivatives; triazine derivatives; phenylbenzotriazole derivatives; benzalmalonate derivatives, especially those mentioned in patent US 5 624 663; phenylbenzimidazole derivatives; imidazolines; 4,4-diarylbutadiene derivatives; bis-benzoazolyl derivatives as described in patents EP 669 323 and US 2 463 264; p-aminobenzoic acid (PABA) derivatives; methylenebis(hydroxyphenylbenzotriazole) derivatives as described in patent applications US 5 237 071, US 5 166 355, GB 2 303 549, DE 197 26 184 and EP 893 119; benzoxazole derivatives as described in patent applications EP 0 832 642, EP 1 027 883, EP 1 300 137 and DE 101 62 844; screening polymers and screening silicones such as those described in particular in patent application WO 93/04665; alpha-alkylstyrene-based dimers, such as those described in patent application DE 198 55 649; 4,4-diarylbutadienes as described in patent applications EP 0 967 200, DE 197 46 654, DE 197 55 649, EP-A-1 008 586, EP 1 133 980 and EP 133 981; merocyanin derivatives such as those described in patent applications WO 04/006 878, WO 05/058 269 and WO 06/032 741; and mixtures thereof.

As examples of organic UV screening agents, mention may be made of those denoted hereinbelow under their INCI name:

### Dibenzoylmethane derivatives:

Butylmethoxydibenzoylmethane, sold under the trade name Parsol 1789 by the company DSM Nutritional Products.

### para-Aminobenzoic acid derivatives:

PABA,
Ethyl PABA,
Ethyl Dihydroxypropyl PABA,
Ethylhexyl Dimethyl PABA sold in particular under the name Escalol 507 by ISP,
Glyceryl PABA,
PEG-25 PABA sold under the name Uvinul P25 by BASF.

### Salicylic derivatives:

Homosalate sold under the name Eusolex HMS by Rona/EM Industries,
Ethylhexyl salicylate sold under the name Neo Heliopan OS by Symrise,
Dipropylene glycol salicylate sold under the name Dipsal by Scher,
TEA Salicylate sold under the name Neo Heliopan TS by Symrise.

### Cinnamic derivatives:

Ethylhexyl methoxycinnamate sold in particular under the trade name Parsol MCX by DSM Nutritional Products,
Isopropyl methoxycinnamate,
Isoamyl methoxycinnamate sold under the trade name Neo Heliopan E 1000 by Symrise,
Cinoxate,
DEA Methoxycinnamate,
Diisopropyl methylcinnamate,
Glyceryl ethylhexanoate dimethoxycinnamate.

### β,β-Diphenylacrylate derivatives:

Octocrylene sold in particular under the trade name Uvinul N539 by BASF,
Etocrylene sold in particular under the trade name Uvinul N35 by BASF.

### Benzophenone derivatives:

Benzophenone-1 sold under the trade name Uvinul 400 by BASF,
Benzophenone-2 sold under the trade name Uvinul D50 by BASF,
Benzophenone-3 or oxybenzone sold under the trade name Uvinul M40 by BASF,
Benzophenone-4 sold under the trade name Uvinul MS40 by BASF,
Benzophenone-5,
Benzophenone-6 sold under the trade name Helisorb 11 by Norquay,
Benzophenone-8 sold under the trade name Spectra-Sorb UV-24 by American Cyanamid,
Benzophenone-9 sold under the trade name Uvinul DS-49 by BASF,
Benzophenone-12,
   n-Hexyl 2-(4-diethylamino-2-hydroxybenzoyl)benzoate sold under the trade name Uvinul A+, or in the form of a mixture with octyl methoxycinnamate under the trade name Uvinul A+B by BASF,
   1,1'-(1,4-Piperazinediyl)bis[1-[2-[4-(diethylamino)-2-hydroxybenzoyl]phenyl]methanone (CAS 919803-06-8).

### Benzylidenecamphor derivatives:

3-Benzylidenecamphor manufactured under the name Mexoryl SD by Chimex,
4-Methylbenzylidenecamphor sold under the name Eusolex 6300 by Merck,
Benzylidenecamphorsulphonic acid manufactured under the name Mexoryl SL by Chimex,
Camphor Benzalkonium Methosulfate manufactured under the name Mexoryl SO by Chimex,
Terephthalylidenedicamphorsulphonic acid manufactured under the name Mexoryl SX by Chimex,
Polyacrylamidomethylbenzylidenecamphor manufactured under the name Mexoryl SW by Chimex.

### Phenylbenzimidazole derivatives:

Phenylbenzimidazole sulphonic acid sold in particular under the trade name Eusolex 232 by Merck,
Disodium phenyl dibenzimidazole tetrasulphonate sold under the trade name Neo Heliopan AP by Symrise.

### Phenylbenzotriazole derivatives:

Drometrizole trisiloxane sold under the name Silatrizole by Rhodia Chimie, Methylenebis(benzotriazolyl)tetramethylbutylphenol sold in solid form under the trade name MIXXIM BB/100 by Fairmount Chemical, or in micronized form as an aqueous dispersion under the trade name Tinosorb M by Ciba Specialty Chemicals.

### Triazine derivatives:

Bis(ethylhexyloxyphenol)methoxyphenyltriazine sold under the trade name Tinosorb S by Ciba Geigy,
Ethylhexyl triazone sold in particular under the trade name Uvinul T150 by BASF,
Diethylhexyl Butamido Triazone sold under the trade name Uvasorb HEB by Sigma 3V,
2,4,6-Tris(diisobutyl 4'-aminobenzalmalonate)-s-triazine,
2,4,6-Tris(dineopentyl 4'-aminobenzalmalonate)-s-triazine,
2,4-Bis(dineopentyl 4'-aminobenzalmalonate)-6-(n-butyl 4'-amino benzoate)-s-triazine,
2,4-Bis(n-butyl 4'-aminobenzoate)-6-[(3-{1,3,3,3-tetramethyl-1-[(trimethylsilyl)oxy]-disiloxanyl}propyl)amino]-s-triazine,
the symmetrical triazine screening agents described in patent US 6 225 467, patent application WO 2004/085412 (see compounds 6 and 9) or the document "Symmetrical Triazine Derivatives" IP.COM Journal, IP.COM INC West Henrietta, NY, US (20 September 2004), especially 2,4,6-tris(biphenyl)-1,3,5-triazines (in particular 2,4,6-tris(biphenyl-4-yl)-1,3,5-triazine) and 2,4,6-tris(terphenyl)-1,3,5-triazine which is also mentioned in Beiersdorf patent applications WO 06/035000, WO 06/034982, WO 06/034991, WO 06/035007, WO 2006/034992 and WO 2006/034985.

### Anthranilic derivatives:

Menthyl anthranilate sold under the trade name Neo Heliopan MA by Symrise.

### Imidazoline derivatives:

Ethylhexyl Dimethoxybenzylidene Dioxoimidazoline Propionate.

### Benzalmalonate derivatives:

Dineopentyl 4'-methoxybenzalmalonate,
Polyorganosiloxane containing benzalmalonate functions, for instance Polysilicone-15, sold under the trade name Parsol SLX by Hoffmann LaRoche.

### 4,4-Diarylbutadiene derivatives:

1,1-dicarboxy(2,2'-dimethylpropyl)-4,4-diphenylbutadiene.

### Benzoxazole derivatives:

2,4-bis[5-(1-dimethylpropyl)benzoxazol-2-yl(4-phenyl)imino]-6-(2-ethylhexyl)imino-1,3,5-triazine sold under the name Uvasorb K2A by Sigma 3V,
and mixtures thereof.

The preferential organic screening agents are selected from:
Butylmethoxydibenzoylmethane,
Ethylhexyl methoxycinnamate,
Homosalate which corresponds to homomenthyl salicylate,
Ethylhexyl salicylate,
Octocrylene,
Phenylbenzimidazole sulphonic acid,
Benzophenone-3,
Benzophenone-4,
Benzophenone-5,
n-Hexyl 2-(4-diethylamino-2-hydroxybenzoyl)benzoate,
1,1'-(1,4-Piperazinediyl)bis[1-[2-[4-(diethylamino)-2-hydroxybenzoyl]phenyl]methanone,
4-Methylbenzylidenecamphor,
Terephthalylidenedicamphorsulphonic acid,
Disodium Phenyl Dibenzimidazole Tetrasulphonate,
Ethylhexyl triazone,
Bis(ethylhexyloxyphenol)methoxyphenyltriazine,
Diethylhexyl butamido triazone,
2,4,6-Tris(biphenyl-4-yl)-1,3,5-triazine,
2,4,6-tris(Dineopentyl 4'-aminobenzalmalonate)-s-triazine
2,4,6-Tris(diisobutyl 4'-aminobenzalmalonate)-s-triazine,
2,4-Bis(dineopentyl 4'-aminobenzalmalonate)-6-(n-butyl 4'-amino benzoate)-s-triazine,
2,4-Bis(n-butyl 4'-aminobenzoate)-6-[(3-{1,3,3,3-tetramethyl-1-[(trimethylsilyl)oxy]-disiloxanyl}propyl)amino]-s-triazine, Methylenebis(benzotriazolyl)tetramethylbutylphenol,
Drometrizole Trisiloxane,
Polysilicone-15,
Dineopentyl 4'-methoxybenzalmalonate,
1,1-dicarboxy(2,2'-dimethylpropyl)-4,4-diphenylbutadiene,
2,4-bis[5-(1-dimethylpropyl)benzoxazol-2-yl(4-phenyl)imino]-6-(2-ethylhexyl)imino-1,3,5-triazine,
and mixtures thereof.

The mineral UV screening agents used in accordance with the present invention are metal oxide pigments. More preferentially, the mineral UV screening agents of the invention are metal oxide particles with a mean elemental particle size of less than or equal to 500 nm, more preferentially between 5 nm and 500 nm and even more preferentially between 10 nm and 100 nm, and preferentially between 15 and 50 nm.

They may be selected in particular from titanium oxide, zinc oxide, iron oxide, zirconium oxide and cerium oxide, or mixtures thereof.

Such coated or uncoated metal oxide pigments are described in particular in patent application EP-A-0 518 773. Commercial pigments that may be mentioned include the products sold by the companies Kemira, Tayca, Merck and Degussa.

The metal oxide pigments may be coated or uncoated.

The coated pigments are pigments that have undergone one or more surface treatments of chemical, electronic, mechanochemical and/or mechanical nature with compounds such as amino acids, beeswax, fatty acids, fatty alcohols, anionic surfactants, lecithins, sodium, potassium, zinc, iron or aluminium salts of fatty acids, metal alkoxides (of titanium or aluminium), polyethylene, silicones, proteins (collagen, elastin), alkanolamines, silicon oxides, metal oxides or sodium hexametaphosphate.

The coated pigments are more particularly titanium oxides that have been coated:
- with silica, such as the product Sunveil from the company Ikeda,
- with silica and iron oxide, such as the product Sunveil F from the company Ikeda,
- with silica and alumina, such as the products Microtitanium Dioxide MT 500 SA and Microtitanium Dioxide MT 100 SA from the company Tayca and Tioveil from the company Tioxide,
- with alumina, such as the products Tipaque TTO-55 (B) and Tipaque TTO-55 (A) from the company Ishihara and UVT 14/4 from the company Kemira,
- with alumina and aluminium stearate, such as the products Microtitanium Dioxide MT 100 T, MT 100 TX, MT 100 Z and MT-01 from the company Tayca, the products Solaveil CT-10 W and Solaveil CT 100 from the company Uniqema and the product Eusolex T-AVO from the company Merck,
- with silica, alumina and alginic acid, such as the product MT-100 AQ from the company Tayca,
- with alumina and aluminium laurate, such as the product Microtitanium Dioxide MT 100 S from the company Tayca,
- with iron oxide and iron stearate, such as the product Microtitanium Dioxide MT 100 F from the company Tayca,
- with zinc oxide and zinc stearate, such as the product BR351 from the company Tayca,
- with silica and alumina and treated with a silicone, such as the products Microtitanium Dioxide MT 600 SAS, Microtitanium Dioxide MT 500 SAS or Microtitanium Dioxide MT 100 SAS from the company Tayca,
- with silica, alumina and aluminium stearate and treated with a silicone, such as the product STT-30-DS from the company Titan Kogyo,
- with silica and treated with a silicone, such as the product UV-Titan X 195 from the company Kemira,
- with alumina and treated with a silicone, such as the products Tipaque TTO-55 (S) from the company Ishihara or UV Titan M 262 from the company Kemira,
- with triethanolamine, such as the product STT-65-S from the company Titan Kogyo,
- with stearic acid, such as the product Tipaque TTO-55 (C) from the company Ishihara,
- with sodium hexametaphosphate, such as the product Microtitanium Dioxide MT 150 W from the company Tayca.
- TiO₂ treated with octyltrimethylsilane, sold under the trade name T 805 by the company Degussa Silices,
- TiO₂ treated with a polydimethylsiloxane, sold under the trade name 70250 Cardre UF TiO2SI3 by the company Cardre,
- anatase/rutile TiO₂ treated with a polydimethylhydrogenosiloxane, sold under the trade name Microtitanium Dioxide USP Grade Hydrophobic by the company Color Techniques.

The uncoated titanium oxide pigments are sold, for example, by the company Tayca under the trade names Microtitanium Dioxide MT 500 B or Microtitanium Dioxide MT 600 B, by the company Degussa under the name P 25, by the company Wackherr under the name Transparent titanium oxide PW, by the company Miyoshi Kasei under the name UFTR, by the company Tomen under the name ITS and by the company Tioxide under the name Tioveil AQ.

The uncoated zinc oxide pigments are for example:
- those sold under the name Z-Cote by the company Sunsmart;
- those sold under the name Nanox by the company Elementis;
- those sold under the name Nanogard WCD 2025 by the company Nanophase Technologies.

The coated zinc oxide pigments are for example:
- those sold under the name Zinc Oxide CS-5 by the company Toshibi (ZnO coated with polymethylhydrogenosiloxane);
- those sold under the name Nanogard Zinc Oxide FN by the company Nanophase Technologies (as a 40% dispersion in Finsolv TN, C12-C15 alkyl benzoate);
- those sold under the name Daitopersion ZN-30 and Daitopersion ZN-50 by the company Daito (dispersions in cyclopolymethylsiloxane/oxyethylenated polydimethylsiloxane, containing 30% or 50% of nanozinc oxides coated with silica and polymethylhydrogenosiloxane);
- those sold under the name NFD Ultrafine ZnO by the company Daikin (ZnO coated with perfluoroalkyl phosphate and copolymer based on perfluoroalkylethyl as a dispersion in cyclopentasiloxane);
- those sold under the name SPD-Z1 by the company Shin-Etsu (ZnO coated with silicone-grafted acrylic polymer, dispersed in cyclodimethylsiloxane);
- those sold under the name Escalol Z100 by the company ISP (alumina-treated ZnO dispersed in an ethylhexyl methoxycinnamate/PVP-hexadecene/methicone copolymer mixture);
- those sold under the name Fuji ZnO-SMS-10 by the company Fuji Pigment (ZnO coated with silica and polymethylsilsesquioxane);
- those sold under the name Nanox Gel TN by the company Elementis (ZnO dispersed at a concentration of 55% in C₁₂-C₁₅ alkyl benzoate with hydroxystearic acid polycondensate).

The uncoated cerium oxide pigments may be, for example, those sold under the name Colloidal Cerium Oxide by the company Rhône-Poulenc.

The uncoated iron oxide pigments are sold, for example, by the company Arnaud under the names Nanogard WCD 2002 (FE 45B), Nanogard Iron FE 45 BL AQ, Nanogard FE 45R AQ and Nanogard WCD 2006 (FE 45R) or by the company Mitsubishi under the name TY-220.

The coated iron oxide pigments are sold, for example, by the company Arnaud under the names Nanogard WCD 2008 (FE 45B FN), Nanogard WCD 2009 (FE 45B 556), Nanogard FE 45 BL 345 and Nanogard FE 45 BL or by the company BASF under the name Transparent Iron Oxide.

Mention may also be made of mixtures of metal oxides, in particular of titanium dioxide and of cerium dioxide, including the equal-weight mixture of titansium dioxide and cerium dioxide coated with silica, sold by the company Ikeda under the name Sunveil A, and also the mixture of titanium dioxide and zinc dioxide coated with alumina, silica and silicone, such as the product M 261 sold by the company Kemira, or coated with alumina, silica and glycerol, such as the product M 211 sold by the company Kemira.

According to the invention, coated or uncoated titanium oxide pigments are particularly preferred.

The cosmetic composition according to the invention may also comprise one or more compounds that are structurally close to the compounds constituting all or part of sebum, and in particular compounds such as squalene.

The cosmetic composition may also comprise one or more compounds selected from fatty substances which are incapable of reacting or barely reactive with ozone.

In particular, such compounds can be selected from saturated fatty substances such as squalane.

Of course, a person skilled in the art will take care to select the aforementioned optional additional compound(s) and/or the amounts thereof such that the advantageous properties intrinsically associated with the compositions in accordance with the invention are not, or are not substantially, adversely affected by the envisaged addition(s).

The pH of the composition in accordance with the invention can be adjusted by means of acidifying or basifying agents.

Among the acidifying agents that may be mentioned, for example, are mineral or organic acids, for instance hydrochloric acid, orthophosphoric acid or sulphuric acid, carboxylic acids, for instance acetic acid, tartaric acid, citric acid and lactic acid, and sulphonic acids.

Among the basifying agents, examples that may be mentioned include aqueous ammonia, alkali metal carbonates, alkanolamines, such as mono-, di- and triethanolamines and derivatives thereof, sodium hydroxide, potassium hydroxide and the compounds of formula (VI) below: 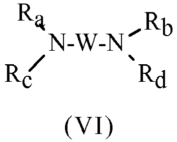 in which W is a propylene residue optionally substituted with a hydroxyl group or a C₁-C₄ alkyl radical; and Rₐ, R_{b}, R_{c} and R_{d}, which may be identical or different, represent a hydrogen atom or a C₁-C₄ alkyl or C₁-C₄ hydroxyalkyl radical.

Preferably, the cosmetic composition comprises one or more basifying agents selected from alkanolamines, in particular triethanolamine, and sodium hydroxide.

The pH of the composition in accordance with the invention is generally between 3 and 12 approximately, preferably between 5 and 11 approximately and even more particularly from 7 to 9.5.

In accordance with the present invention, the carbonyl compound(s) B may be compounds having one or more aldehyde functions formed following the addition reaction which takes place between one or more constituents of sebum and ozone.

The compound(s) B comprising one or more aldehyde functions may be simple aldehydes, conjugated aldehydes, aromatic aldehydes such as benzaldehydes and activated ketones, in particular activated with a withdrawing group, or conjugation functions.

The sebum corresponds to the secretion by the sebaceous glands of a lipid film which is found at the surface of the skin.

Thus, the sebum contains a lipid concentration that can range up to 96% by weight. In particular, the sebum contains approximately 43% by weight of glycerides, 16% by weight of free fatty acids, 25% by weight of wax and 12% by weight of squalene.

According to one specific embodiment, the compound(s) B may be compounds having one or more aldehyde functions resulting from the reaction between squalene and ozone.

Even more particularly, the compound(s) B having one or more aldehyde functions may be, for example, 4-oxopentanal, nonanal or nonenal.

According to one embodiment, the application of the cosmetic composition according to the invention is not followed by a step of rinsing the surface of the keratin material.

According to another embodiment, the application of the cosmetic composition is followed by drying at ambient temperature and then by rinsing of the surface of the keratin material.

The drying may be performed immediately after the application or after a leave-on time that may range from 1 minute to 30 minutes. Preferably, the drying time is sufficiently long for the cosmetic composition according to the invention to dry on the skin.

Preferably, the cosmetic composition according to the invention is not rinsed off.

The cosmetic composition may be prepared according to techniques that are well known to those skilled in the art. It may in particular be in the form of a simple or complex emulsion (O/W, W/O, O/W/O or W/O/W) such as a cream, a milk or a cream-gel; in the form of an aqueous gel; or in the form of a lotion.

The cosmetic composition according to the invention may be used, for example, as a makeup product, a hair or scalp treatment product such as shampoos or care compositions, or a care and/or antisun protection product for the face and/or the body, of liquid to semi-liquid consistency, such as milks, more or less rich creams, cream-gels and pastes. The cosmetic composition may optionally be packaged in the form of wipes or as an aerosol, and be in the form of a foam or a spray.

Preferably, the cosmetic treatment process according to the invention consists in applying the compound(s) A described above to the skin.

In particular, the cosmetic composition may be used for treating clothing, shoes or any other object that may be in contact with the body.

The present invention also relates to a process for treating fabrics, leathers or any material in contact with a keratin material, comprising the application, to a surface of the fabric, of the leather or of said material, of a composition as defined above.

The cosmetic treatment process according to the invention can be carried out daily or less regularly.

The present invention thus makes it possible to block the decomposition products resulting from the action of ozone on the skin.

The examples that follow serve to illustrate the invention without, however, being limiting in nature.

### EXAMPLES

### Example 1:

A cosmetic composition containing 10% by weight of APTES sold under the name Dow Corning is prepared. The pH of the composition is brought to 9 by adding lactic acid.

The cosmetic composition is then applied to the skin in a proportion of 4 mg per cm² over the whole of the body, and then left to dry.

Nonanal, which is an aldehyde that can be formed following the action of ozone on sebum, is then applied to the skin.

It is noted that the nonanal is trapped by the APTES in the skin.

The studies on sections demonstrate that the blocking has taken place just below the surface of the skin, in the stratum corneum.

### Example 2:

An aqueous cosmetic composition containing 1.4% by weight of active materials of a vinylformamide/vinylformamine copolymer sold under the name Lupamine 9030 by the company BASF is prepared.

It is applied to the skin in a proportion of 4 mg per cm² over the whole of the body, and then left to dry.

Nonanal, which is a compound structurally close to those which are formed following the action of ozone on sebum, is then applied to the skin.

It is noted that the nonanal is trapped on the skin.

When the skin perspires, the layer formed by the vinylformamide/vinylformamine copolymer is submerged, but the compound remains, by virtue of its substantivity, on the skin. The nonanal is not released, despite the presence of perspiration.

## Claims

1. Compounds A having one or more nucleophilic functions F_{A} capable of reacting with one or more carbonyl compounds B resulting from the reaction between one or more compounds constituting sebum and ozone, for their use as a dermatological agent for preventing and/or treating skin disorders induced by the carbonyl compounds B.

2. Compounds A for use according to claim 1, **characterized in that** they are capable of condensing in situ in the skin, in particular the superficial layers of the skin

3. Compounds A for use according to claim 1 or 2, **characterized in that** they are selected from organic compounds comprising from 1 to 3 silicon atoms, and at least two hydroxyl or hydrolysable groups.

4. Compounds A for use according to claim 3, **characterized in that** the nucleophilic function F_{A} of the organic silicon compound is an amine function, preferably a primary amine.

5. Compounds A for use according to any one of Claims 1 to 4, **characterized in that** they are selected from the organic silicon compounds of formula: 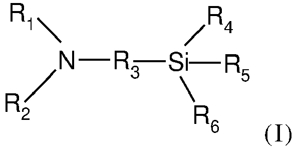 in which:
R4 represents a halogen or a group OR' or R'₁;
R₅ represents a halogen or a group OR" or R'₂;
R6 represents a halogen or a group OR'" or R'₃;
R₁, R₂, R₃, R', R", R''', R'₁, R'₂ and R'₃ represent, independently of one another, a saturated or unsaturated, linear or branched hydrocarbon-based group, optionally bearing additional chemical groups, R₁, R₂, R', R" and R'" also possibly denoting hydrogen, and at least two of the groups R₄, R₅ and R₆ denoting, respectively, OR', OR" and OR''', at least two of the groups R', R" and R'" being other than hydrogen.

6. Compounds A for use according to any one of Claims 1 to 5, **characterized in that** they are selected from the organic silicon compounds of formula (III): 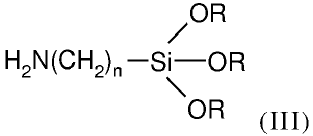 in which the radicals R, which may be identical or different, are selected from C₁-C₆ alkyl radicals and n is an integer from 1 to 6, preferably from 2 to 4.

7. Compounds A for use according to any one of Claims 1 to 6, **characterized in that** they are γ-aminopropyltriethoxysilane (APTES) or a derivative thereof.

8. Compounds A for use according to Claim 1, **characterized in that** they are capable of forming an external capture layer on the surface of the skin, in particular they comprise at least one nucleophilic function F_{A} of the non-quaternized amine, and preferably primary amine, type.

9. Compounds A for use according to Claim 8, **characterized in that** they are selected from polymers bearing pendant amino groups, more particularly polymers bearing at least 10%, relative to the monomeric entities, of amino functions.

10. Compounds A for use according to Claim 8 or 9, **characterized in that** they are selected from the vinylformamide/vinylformamine copolymers comprising:
- from 10 to 95 mol% of units of the following formula (IV): 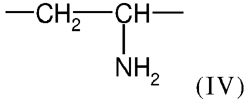 and from 90 to 5 mol% of units of the following formula (V):

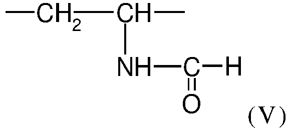

11. Compounds A for use according to Claim 10, **characterized in that** the vinylformamide/vinylformamine copolymer(s) comprise(s) from 10 to 60 mol%, preferably from 20 to 40 mol%, of units of formula (IV).

12. Compounds A for use according to any one of Claims 8 to 11, **characterized in that** the vinylformamide/vinylformamine copolymer(s) comprise(s) one or more additional monomer units, the latter representing less than 20 mol% of the copolymer.

13. Compounds A for use according to any one of the preceding claims, **characterized in that** the compound(s) B is (are) compounds having one or more aldehyde functions resulting from the reaction between squalene and ozone.

14. Compounds A for use according to any one of the preceding claims, as a soothing agent for preventing and/or treating discomforting reactions of keratin materials induced by said carbonyl compounds B.

15. Compounds A for use according to any one of Claims 1 to 13, as an agent for reducing or even eliminating the unpleasant body odours produced by said carbonyl compounds B.

## Patentansprüche

1. Verbindungen A mit einer oder mehreren nukleophilen Funktionen F_{A}, die dazu fähig sind, mit einer oder mehreren Carbonylverbindungen B, die aus der Umsetzung zwischen einer oder mehreren Verbindungen, die Bestandteile von Hauttalg sind, und Ozon hervorgehen, zu reagieren, zur Verwendung als dermatologisches Mittel zur Prävention und/oder Behandlung von durch die Carbonylverbindungen B induzierten Hautkrankheiten.

2. Verbindungen A zur Verwendung nach Anspruch 1, **dadurch gekennzeichnet, dass** sie dazu fähig sind, in situ in die Haut, insbesondere in die oberen Schichten der Haut, zu kondensieren.

3. Verbindungen A zur Verwendung nach Anspruch 1 oder 2, **dadurch gekennzeichnet, dass** sie aus organischen Verbindungen mit 1 bis 3 Siliziumatomen und mindestens zwei Hydroxylgruppen bzw. hydrolysierbare Gruppen ausgewählt sind.

4. Verbindungen A zur Verwendung nach Anspruch 3, **dadurch gekennzeichnet, dass** es sich bei der nukleophilen Funktion F_{A} der organischen Siliziumverbindung um eine Aminfunktion, vorzugsweise ein primäres Amin, handelt.

5. Verbindungen A zur Verwendung nach einem der Ansprüche 1 bis 4, **dadurch gekennzeichnet, dass** sie ausgewählt sind aus den organischen Siliziumverbindungen der Formel: 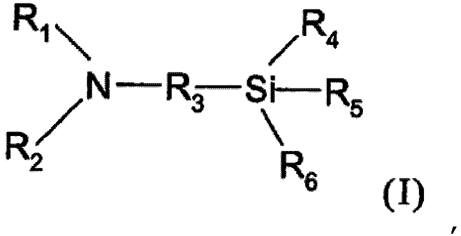 in welcher:
R₄ für ein Halogen oder eine Gruppe OR' oder R'₁ steht,
R₅ für ein Halogen oder eine Gruppe OR" oder R'₂ steht,
R₆ für ein Halogen oder eine Gruppe OR''' oder R'₃ steht,
R₁, R₂, R₃, R', R", R''', R'₁, R'₂ und R'₃ unabhängig voneinander für eine gesättigte oder ungesättigte, geradkettige oder verzweigte Gruppe auf Kohlenwasserstoffbasis stehen, die gegebenenfalls zusätzliche chemische Gruppen trägt, wobei R₁, R₂, R', R" und R''' außerdem möglicherweise für Wasserstoff stehen und mindestens zwei der Gruppen R₄, R₅ und R₆ für OR', OR" bzw. OR''' stehen, wobei mindestens zwei der Gruppen R', R" und R''' nicht für Wasserstoff stehen.

6. Verbindungen A zur Verwendung nach einem der Ansprüche 1 bis 5, **dadurch gekennzeichnet, dass** sie ausgewählt sind aus den organischen Siliziumverbindungen der Formel (III): 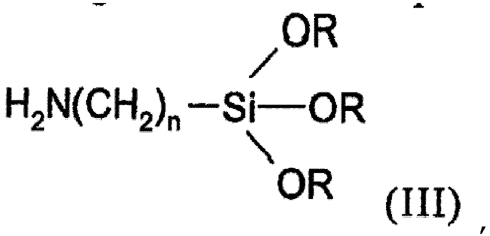 in welcher die Reste R, die gleich oder verschieden sein können, aus C₁-C₆-Alkylresten ausgewählt sind und n für eine ganze Zahl von 1 bis 6, vorzugsweise 2 bis 4, steht.

7. Verbindungen A zur Verwendung nach einem der Ansprüche 1 bis 6, **dadurch gekennzeichnet, dass** es sich bei ihnen um γ-Aminopropyltriethoxysilan (APTES) oder ein Derivat davon handelt.

8. Verbindungen A zur Verwendung nach Anspruch 1, **dadurch gekennzeichnet, dass** sie dazu fähig sind, auf der Oberfläche der Haut eine externe Abfangschicht zu bilden, und sie insbesondere mindestens eine nukleophile Funktion F_{A} des nichtquaternisierten Amintyps und vorzugsweise primären Amintyps umfassen.

9. Verbindungen A zur Verwendung nach Anspruch 8, **dadurch gekennzeichnet, dass** sie aus Polymeren, die anhängige Aminogruppen tragen, besonders bevorzugt Polymeren, die bezogen auf die monomeren Einheiten mindestens 10 % Aminofunktionen tragen, ausgewählt sind.

10. Verbindungen A zur Verwendung nach Anspruch 8 oder 9, **dadurch gekennzeichnet, dass** sie aus den Vinylformamid-Vinylformamin-Copolymeren ausgewählt sind, die Folgendes umfassen:
- 10 bis 95 Mol-% Einheiten der folgenden Formel (IV) : 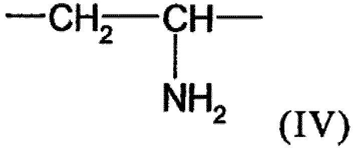 und
90 bis 5 Mol-% Einheiten der folgenden Formel (V):

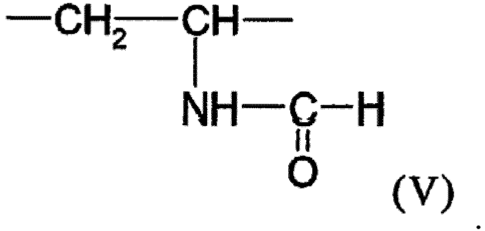

11. Verbindungen A zur Verwendung nach Anspruch 10, **dadurch gekennzeichnet, dass** der/die Vinylformamid-Vinylformamin-Copolymer(e) 10 bis 60 Mol-%, vorzugsweise 20 bis 40 Mol-%, Einheiten der Formel (IV) umfasst/umfassen.

12. Verbindungen A zur Verwendung nach einem der Ansprüche 8 bis 11, **dadurch gekennzeichnet, dass** das/die Vinylformamid-Vinylformamin-Copolymer(e) ein oder mehrere zusätzliche Monomereinheiten umfasst/umfassen, wobei letztere weniger als 20 Mol-% des Copolymers ausmachen.

13. Verbindungen A zur Verwendung nach einem der vorhergehenden Ansprüche, **dadurch gekennzeichnet, dass** die Verbindung(en) B Verbindung mit einer oder mehreren aus der Umsetzung von Squalen mit Ozon hervorgehenden Aldehydfunktionen ist/sind.

14. Verbindungen A zur Verwendung nach einem der vorhergehenden Ansprüche als linderndes Mittel zur Prävention und/oder Behandlung von unbehaglichen Reaktionen von Keratinmaterialien, die durch die Carbonylverbindungen B induziert werden.

15. Verbindungen A zur Verwendung nach einem der Ansprüche 1 bis 13 als ein Mittel zum Reduzieren oder sogar Eliminieren der von den Carbonylverbindungen B hervorgerufenen unangenehmen Körpergerüche.

## Revendications

1. Composés A ayant une ou plusieurs fonctions nucléophiles F_{A} susceptibles de réagir avec un ou plusieurs composés carbonylés B issus de la réaction entre un ou plusieurs composés constituant le sébum et l'ozone pour leur utilisation en tant qu'agent dermatologique pour la prévention et/ou le traitement des désordres cutanés induits par les composés carbonylés B.

2. Composés A pour leur utilisation selon la revendication 1, **caractérisés en ce que** le ou les composés A sont aptes à se condenser in situ dans peau, notamment les couches superficielles de la peau.

3. Composés A pour leur utilisation selon la revendication 1 ou 2, **caractérisés en ce qu'**ils sont choisis parmi les composés organiques comprenant de 1 à 3 atomes de silicium, et au moins deux groupes hydroxyles ou hydrolysables.

4. Composés A pour leur utilisation selon la revendication 3, **caractérisés en ce que** la fonction nucléophile F_{A} du composé organique du silicium est une fonction amine, de préférence une amine primaire.

5. Composés A pour leur utilisation selon l'une quelconque des revendications 1 à 4, **caractérisés en ce qu'**ils sont choisis parmi les composés organiques du silicium de formule : 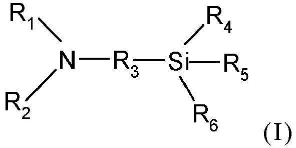 dans laquelle :
R₄ représente un halogène, un groupe OR' ou R'1 ;
R₅ représente un halogène, un groupe OR" ou R'2 ;
R₆ représente un halogène, un groupe OR''' ou R'3 ;
R₁, R₂, R₃, R', R", R"', R'₁, R'₂, R'₃ représentent, indépendamment les uns des autres, un groupe hydrocarboné saturé ou insaturé, linéaire ou ramifié, portant éventuellement des groupes chimiques supplémentaires, R₁, R₂, R', R" et R'" pouvant en outre désigner l'hydrogène, et deux au moins des groupes R₄, R₅ et R₆ désignant respectivement OR', OR" et OR''', deux au moins des groupes R', R" et R'" étant différents de l'hydrogène.

6. Composés A pour leur utilisation selon l'une quelconque des revendications 1 à 5, **caractérisés en ce qu'**ils sont choisis parmi les composés organiques du silicium de formule (III): 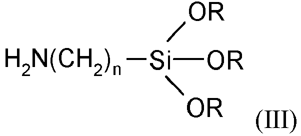 dans laquelle les radicaux R, identiques ou différents, sont choisis parmi les radicaux alkyle en C₁-C₆ et n est un nombre entier de 1 à 6, de préférence de 2 à 4.

7. Composés A pour leur utilisation selon l'une quelconque des revendications 1 à 6, **caractérisés en ce qu'**ils sont le γ-aminopropyl triéthoxysilane (APTES) ou un de ses dérivés.

8. Composés A pour leur utilisation selon la revendication 1, **caractérisés en ce qu'**ils sont aptes à former une couche de capture externe sur la surface de la peau, en particulier le ou les composés A comprennent au moins une fonction F_{A} nucléophile du type amine non quaternisée et de préférence amine primaire.

9. Composés A pour leur utilisation selon la revendication 8, **caractérisés en ce qu'**ils sont choisis parmi les polymères portant des groupes aminés pendants, plus particulièrement les polymères portant au moins 10% par rapport aux entités monomériques de fonctions aminées.

10. Composés A pour leur utilisation selon la revendication 8 ou 9, **caractérisés en ce qu'**ils sont choisis parmi les copolymères vinylformamide / vinylformamine comprenant :
- de 10 à 95 % en moles de motifs de formule suivante (IV) : 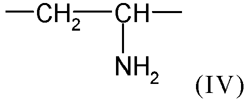 et de 90 à 5 % en moles de motifs de formule suivante (V):

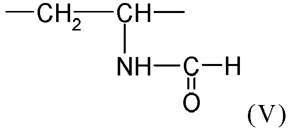

11. Composés A pour leur utilisation selon la revendication 10, **caractérisés en ce que** le ou les copolymères vinylformamide / vinylformamine comprennent de 10 à 60% en moles, de préférence 20 à 40% en moles de motif de formule (IV).

12. Composés A pour leur utilisation selon l'une quelconque des revendications 8 à 11, **caractérisés en ce que** le ou les copolymères vinylformamide / vinylformamine comprennent un ou plusieurs motifs monomères additionnels, ces derniers représentant moins de 20% en moles du copolymère.

13. Composés A pour leur utilisation selon l'une quelconque des revendications précédentes, **caractérisés en ce que** le ou les composés B sont des composés ayant une ou plusieurs fonctions aldéhydes issus de la réaction entre le squalène et l'ozone.

14. Composés A pour leur utilisation tels que définis selon l'une quelconque des revendications précédentes comme agent apaisant pour prévenir et/ou traiter des réactions d'inconforts des matières kératiniques induites par lesdits composés carbonylés B.

15. Composés A pour leur utilisation tels que définis selon l'une quelconque des revendications 1 à 13 comme agent permettant de réduire voire d'éliminer les mauvaises odeurs corporelles produites par lesdits composés carbonylés B.
